# EUROPEAN PATENT SPECIFICATION

(11) **EP 2 268 259 B1**
(45) Date of publication and mention of the grant of the patent: **15.11.2017**
(21) Application number: 09739808.5
(22) Date of filing: 30.04.2009
(51) Int. Cl.: A61K 8/49, A61K 8/67, A61K 8/42, A61Q 5/00, A61Q 5/12, A61Q 5/02, A61Q 5/06, A61K 8/04

(54) **METHODS FOR PREVENTING OXIDATIVE DAMAGE OF HAIR ATTRIBUTED TO THE PRESENCE OF FUNGI IN THE HAIR FOLLICLE**
VERFAHREN ZUR VERHINDERUNG VON OXIDATIONSSCHÄDIGUNGEN DER HAARE AUFGRUND DER ANWESENHEIT VON FUNGI IM HAARFOLLIKEL
PROCÉDÉ POUR PRÉVENIR LES DOMMAGES OXYDATIFS DES CHEVEUX ATTRIBUÉS À LA PRÉSENCE DE CHAMPIGNONS PARASITES DANS LE FOLLICULE PILEUX

(30) Priority: 30.04.2008 US 49202 P
(43) Date of publication of application: 05.01.2011
(73) Proprietor: The Procter & Gamble Company, Cincinnati, OH 45202 (US)
(72) Inventor: DAWSON, Thomas, Larry, Jr., Hamilton OH 45011 (US); YOUNGQUIST, Robert, Scott, Mason OH 45040 (US); FISHER, Brian, Keith, Cincinnati OH 45249 (US)
(74) Representative: Briatore, Andrea
(86) International application number: PCT/US2009/042317
(87) International publication number: WO 2009/135006

(56) References cited:
- WO-A1-01/06997
- WO-A2-03/015734
- US-A- 5 877 209
- US-A1- 2008 059 313
- DATABASE GNPD [Online] Mintel International Group; February 2008 (2008-02), "Caffeine Shampoo for colour Treated and Stressed Hair", XP002627646, Database accession no. 865596
- DATABASE GNPD [Online] Mintel International Group Ltd.; February 2007 (2007-02), "Hair masque", XP002627645, Database accession no. 654815
- DATABASE gnpd [Online] Mintel International Group; January 2007 (2007-01), "Anti-static weightless mist", XP002627647, Database accession no. 645225
- DATABASE gnpd [Online] January 2007 (2007-01), "Anti-dandruff shampoo", XP002627648, Database accession no. 636995
- DATABASE GNPD [Online] Mintel International Group; March 2007 (2007-03), "Conditioner", XP002627649, Database accession no. 670355
- DATABASE GNPD [Online] Mintel International Group; August 2006 (2006-08), "Pumpkin Rehydrating Shampoo", XP002627650, Database accession no. 575869
- DATABASE gnpd [Online] Mintel International Group Ltd.; August 2006 (2006-08), "ShineLuxe Hair Polish", XP002627651, Database accession no. 571805
- JUNG K ET AL: "Detection of UV induced Free Radicals in Hair and their Prevention by Hair Care Products", SOFW-JOURNAL SEIFEN, OELE, FETTE, WACHSE, VERLAG FUR CHEMISCHE INDUSTRIE, AUGSBURG, DE, vol. 132, no. 7, 1 January 2006 (2006-01-01), pages 32-36, XP002573576, ISSN: 0942-7694
- "Cocoa extract", , 3 May 2005 (2005-05-03), pages 1-22, XP055094416, Retrieved from the Internet: URL:http://www.oryza.co.jp/html/english/pd f/pdf2/Cocoa ver.1.pdf [retrieved on 2013-12-19]
- RAMON GRIMALT: "A Practical Guide to Scalp Disorders", JOURNAL OF INVESTIGATIVE DERMATOLOGY SYMPOSIUM PROCEEDINGS, vol. 12, no. 2, 1 December 2007 (2007-12-01), pages 10-14, XP055209931, ISSN: 1087-0024, DOI: 10.1038/sj.jidsymp.5650048

## Description

### FIELD OF THE INVENTION

Hair care compositions that can be used to prevent oxidative damage to hair, their methods of use, and methods of marketing such compositions.

### BACKGROUND OF THE INVENTION

Many attributes contribute to the appearance of hair considered to be attractive. For instance, undamaged hair is very desirable, whether it be on the scalp, beard, or moustache regions. In contrast, oxidatively damaged hair is not as attractive, and can appear dull, lifeless, and frizzy. Furthermore, oxidatively damaged hair can be more difficult to style and condition, and typically cannot be styled into as many hairstyles, leaving the individual frustrated and with an unkempt appearance. Because of the foregoing problems associated with oxidatively damaged hair, many damaged-haired individuals expend great effort and time on grooming, yet still do not attain their desired hairstyle and appearance. This can lead to frustration and/or lack of confidence in his or her appearance. These problems can be experienced by both female and male consumers. Document XP002627646, Database accession number 865596 describes a caffeine shampoo for colored and stressed hair, document XP002627645, accession number 654815 describes a hair masque designed to renew and repair the hair.

Accordingly, there is a need to provide consumers with a way to prevent hair from experiencing oxidative damage, thus resulting in a more attractive hair and a more attractive hair style.

### BRIEF SUMMARY OF THE INVENTION

The present invention relates to methods for preventing oxidative damage to hair.

In one aspect, the method comprises the use of a hair care composition comprising a follicular fungi reduction agent ("FFRA"). In another aspect, the method comprises topically applying a hair care composition comprising an effective amount of a FFRA to the desired region (e.g., scalp, beard, moustache) of a mammal for the purpose of preventing oxidative damage of hair attributed to the presence of fungi in the hair follicle.

These and other features, aspects, and advantages of the present invention will become evident to those skilled in the art from a reading of the present disclosure.

### BRIEF DESCRIPTION OF THE DRAWINGS

Fig. 1 is an illustration (1a) and micrographs (1b-1c) showing increasing magnification of the hair shaft
Fig. 2 is a graph showing the amplex ultra red fluorescence results from an experiment where *Malassezia* cells on an unsaturated lipophilic substrate were treated with a dye (AMP) which fluoresces when exposed to oxidative substances.
Fig. 3 is a graph showing the amplex fluorescence signal produced when 10µM H₂O₂ is manually added to AMP dye. This figure demonstrates that the signal is quenched by ethoxyquin, and shows the scavenging effect of ethoxyquin in amplex assay (pH=7.4)
Fig. 4 is a graph showing *Malassezia* cell counts after various leave-on tonic treatments comprising FFRAs vs. placebo. The FFRAs demonstrated are: (1) a mixture of niacinamide, caffeine, and panthenol; and (2) a mixture of niacinamide and panthenol. This figure demonstrates that *Malassezia* are removed from the scalp by applying these FFRAs.
Fig. 5 is a graph showing the decreased level of *Malassezia* when a variety of anti-dandruff actives are delivered from a shampoo formulation context after two weeks of usage. From left to right, the bars on the graph represent: 1% SeS2, 1% ZPT, 1% Climbazole, None (Control), 1% Platelet ZPT, 1SeS2, and 1% Ketoconazole.
Fig. 6 is a graph of integrated IR spectral results comparing the reduction in oxidative hair damage obtained using an FFRA tonic formulation (combination of
   caffeine/niacinamide/panthenol) versus a variety of in-market products which claim to improve hair quality but do not containing FFRAs. This shows that the FFRA formulation reduced the amount of oxidative damage to the hair caused by *Malassezia.* From left to right, the bars on the graph represent: Product A, Product B, Product C, Product D, and the technology of this application.

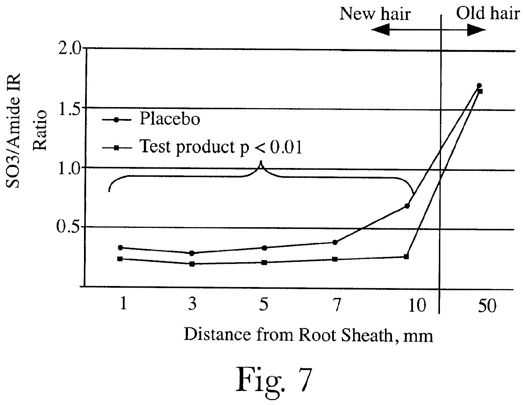

Fig. 7 is a graph showing statistically significantly less oxidative damage was observed with the test product (technology of this application) versus the test product placebo, as discussed in Example 13.

### DETAILED DESCRIPTION OF THE INVENTION

While the specification concludes with the claims particularly pointing and distinctly claiming the invention, it is believed that the present invention will be better understood from the following description.

The present invention provides methods for preventing oxidative damage to hair by applying an effective amount of a FFRA to the desired area; this, in turn, surprisingly leads to prevention of oxidative damage of the hair.

Oxidation breaks chemical bonds between chemical entities and changes their properties. As is relates to hair, oxidation breaks the disulfide bonds of cystine, creating cysteic acid (SO3= as one by-product. This process of oxidation occurs with bleaching and coloring of hair, exposure to UV radiation and by normal daily activities. Oxidation compromises the health of the hair by promoting brittleness, loss of the hair's f-layer, and melanin degradation. The surface of the hair becomes less hydrophobic and more susceptible to water penetration. The prevention of oxidation helps maintain the health of the hair.

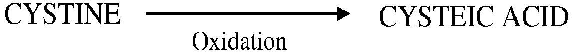

Hair keratin undergoes oxidation by both photochemical (e.g., exposure to UV light and/or atmospheric oxygen) and chemical means (bleaches, permanent waves and/or permanent dyes). Oxidation results in decreased tensile strength of hair due to disulfide bond scission, in color changes due to melanin degradation, and in more easily abraded cuticle due to loss of the f-layer and hydrophobicity. (Martin-K. 4. Infrared and Raman Studies of Skin and Hair: A review of cosmetic spectroscopy. The Internet Journal of Vibrational Spectroscopy. Volume 3, Edition 2 (1999)-Unilever) Cysteic acid residues arise from disulfide bond fission of cystine, the most abundant amino acid found in hair, followed by oxidation. (Strassburger-J and Breuer-MM. Quantitative Fourier transform infrared spectroscopy of oxidize hair. J. Soc. Cosmet. Chem., 36, 61-74 (January/February 1985))

Furthermore, various studies have demonstrated that that oxidative damage results from chemical and physical stress, and cuticle damage results from oxidation. (Takada-K et. al. Influence of Oxidative and/or Reductive Treatment on Human Hair (I): Analysis of Hair-Damage after Oxidative and/or Reductive Treatment. J. Oleo Sci., Vol 52, No. 10, 541-548 (2003)) In addition, such studies have concluded that use of an anti-oxidant decreases cysteic acid and prevents an increase in hair damage. (Takada-K et. al. Influence of Oxidative and/or Reductive Treatment on Human Hair (II): Effect of Hydrophilic Extracts from Rosmarinus officinalis L. on Oxidative and/or Reductive Hair-Damage. J. Oleo Sci., Vol. 52, No. 10, 549-556 (2003)) Chemical and physical stress, as well as environmental stress in daily life, can damage hair. Furthermore, cysteic acid, which is an oxidation product, increases in the hair depending on the amount of stress in daily life.

The cuticles of the hair are covered on the surface by fatty acid f-layer. When these layers are removed by chemical and physical treatment, the hair surface becomes hydrophilic, leading to an increase in cysteic acid and an increase in the frequency of splitting and breaking in the damaged hair. Because the f-layer is responsible for hair shine, damage to or removal of the f-layer results in a reduction in hair shine.

Furthermore, the presence of fungi (e.g., *Malassezia*) in the hair follicle leads to the production of oxidative products, and thus leads to oxidative damage of the hair, even before it emerges from the hair follicle. The fungi also produce enzymes that strip the protective lipid from the surface of the hair which results in increased oxidation of the hair from environmental insults. *See, e.g.*, Jun Xu, et al., (2007) Dandruff-associated Malassezia genomes reveal convergent and divergent virulence traits shared with plant and human fungal pathogens. Proceedings of the National Academy of Science, USA, 104 (47) 18731.

Surprisingly, the present inventors have found that applying an effective amount of a follicular fungi reduction agent ("FFRA") to the desired area can prevent oxidative damage in hair. The FFRA decreases the level of fungi present in the hair follicle and surface, thus leading to less oxidative damage.

Because consumers are not familiar with the use of FFRAs for the purpose of preventing oxidative damage to hair, the present invention also provides methods of marketing that can be advantageously used to help potential consumers appreciate the benefits that they can derive from such a product and/or its method of use. Furthermore, a method of marketing a first composition by comparing it to a second composition that comprises a FFRA is also provided.

All percentages, parts and ratios are based upon the total weight of the hair care compositions of the present invention and all measurements made are at 25°C, unless otherwise specified. All such weights as they pertain to listed ingredients are based on the active level and, therefore, do not include carriers or by-products that may be included in commercially available materials, unless otherwise specified.

As used herein, the term "hair care compositions" are compositions that are applied to the hair and/or the skin underneath the hair, including compositions used to treat or care for the hair. Products contemplated by the phrase "hair care composition" include, but are not limited to liquids, creams, wipes, hair conditioners (rinse-off and leave-on), hair tonics, shampoos, hair colorants, mousses, propellant lotions, emulsions, shave gels, after-shave tonics and lotions, temporary beard hair dyes, and the like.

"Prevent oxidative damage in hair' means the region of hair (e.g., scalp, beard) has less oxidative damage than untreated regions. This is demonstrated when the hair shafts in the subject region of hair are prevented from experiencing oxidation by a statistically significant amount, when a composition of the present invention is used for a result-effective period of time.

"Mammalian hair", as referenced herein, includes hair on any part of the body of a mammal, and can include but is not limited to facial, cranial, or body hair. For instance, it can include hair on the scalp, head, neck, beard, moustache, eyebrows and sideburns hair.

As used herein, the term "follicular fungi reduction agent" or "FFRA" means any material that reduces and/or can reduce the number of fungi present in hair follicles.

The term "topical application," as used herein, means to apply or spread the compositions of the present invention onto the surface of the keratinous tissue from which the hair to be affected grows, and/or to the hair itself.

The term "dermatologically-acceptable," as used herein, means that the compositions or components thereof so described are suitable for use in contact with mammalian keratinous tissue without undue toxicity, incompatibility, instability, allergic response, and the like.

The term "effective amount," as used herein, means an amount of a compound or composition sufficient to decrease the amount of oxidative damage of the hair shaft in the subject region of hair by a statistically significant amount.

The term "result-effective period of time," as used herein, means a period of time sufficient to decrease the amount of oxidative damage of the hair shaft in the subject region of hair by a statistically significant amount.

The term "safe and effective amount," as used herein, means an amount of a compound or composition sufficient to decrease the amount of oxidative damage of the hair shaft in the subject region of hair by a statistically significant amount, but low enough to avoid serious side effects, i.e., to provide a reasonable benefit to risk ratio, within the scope of sound judgment of the skilled artisan.

The term "ambient conditions," as used herein, refers to surrounding conditions under about one atmosphere of pressure, at about 50% relative humidity, and at about 25°C, unless otherwise specified.

### I. Hair Care Compositions comprising FFRAs for Preventing Oxidative Damage

The present invention provides hair care compositions that can be used to prevent oxidative damage in hair attributed to the presence of fungi in the hair follicle. The hair care composition comprises an FFRA. In another embodiment, the hair care composition comprises two or more FFRAs. which comprises a mixture of a xanthine compound, a vitamin B3 compound and a panthenol compound. Preferably, the FFRA(s) is present in an effective amount, more preferably in a safe and effective amount. Optionally, the hair care compositions can comprise a dermatologically-acceptable carrier and/or any desired suitable optional ingredients.

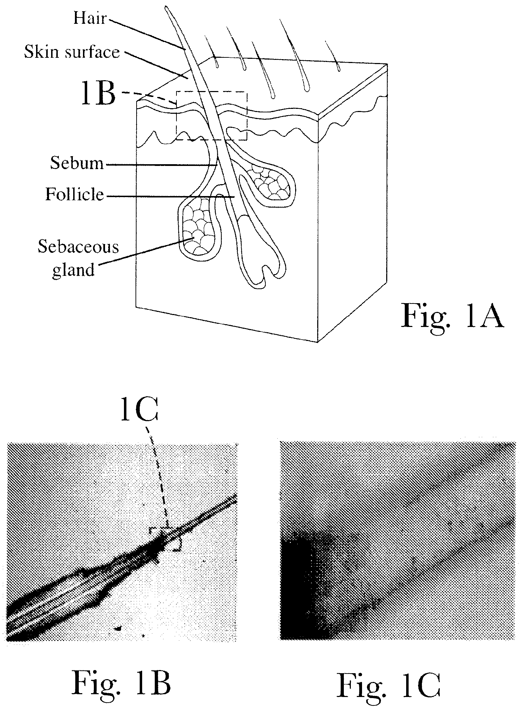

Not wishing to be bound by theory, at least one source of oxidative hair damage can be attributed to the presence of *Malassezia* on the hair in the infundibulum region. As shown in Figure 1 and Table 1, *Malassezia* are present on the hair shaft in the appropriate region of the scalp. Figure 1 presents an illustration and micrographs with increasing magnification of the hair shaft. The focus region is the section of hair shaft that would be present in the infundibulum where the hair is just emerging. A stain is used to visualize the *Malassezia* cells and show that they are attached to the hair shaft in this region.

Table 1 provides further evidence of the presence of *Malassezia* in this region based on fungal cell counts obtained using swab (scalp) and pluck (follicle) extraction assays. The values represent the average number of *Malassezia globosa, Malassezia restricta,* or total fungi cells contained in a sample (n ∼ 100) obtained in each assay. The swab sample is collected by "Swabbing" the surface of a subject's scalp. A pluck sample is a plucked hair with fungi located in the follicle and in very close proximity of the follicle infundibulum. The high density of cells obtained from the pluck assay indicates a fungal affinity for the follicle and follicle infundibulum.

**Table 1. Fungal Cell Counts**

| | Scalp Surface (Swab) cells/cm² | In Follicle, below the Surface (Pluck) cells/cm² |
|---|---|---|
| *M. restricta* on scalp | 211 | 1840 |
| *M. globosa* on scalp | 1186 | 4364 |

The present investigators believe that *Malassezia* produces substances that can oxidatively damage hair. Oxidation of the hair causes damage to the cuticle structure, thereby reducing hair strength and resistance to other damage factors. Although not wishing to be limited by theory, the present investigators hypothesize that *M. globosa* cells cause oxidative damage to hair through one or more mechanisms. For example, *Malassezia* cells secrete aryl alcohol oxidase, which has been demonstrated to produce hydrogen peroxide (See Xu, J., et al; Proceedings of the National Academy of Science, USA, (2007), 104(47), 18731). Exposure to H₂O₂ can cause oxidative damage to hair (see Robbins, C.R.; Chemical and Physical Behavior of Human Hair, 3rd ed., p. 131, Springer-Verlag (New York)).

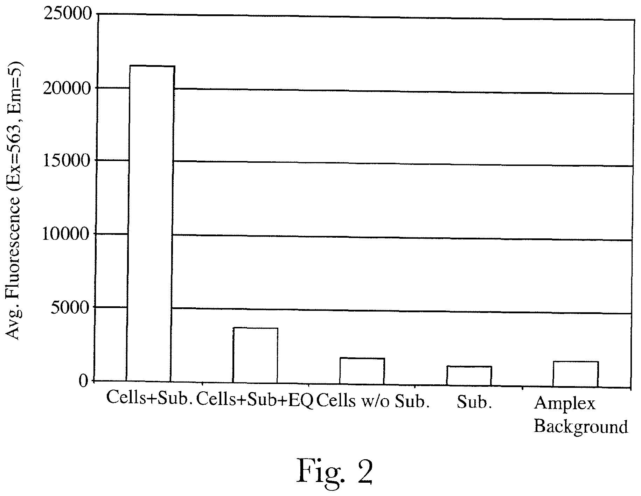

Figure 2 summarizes the results of an experiment where *Malassezia* cells on an unsaturated lipophilic substrate were treated with a dye (AMP) which fluoresces when exposed to oxidative substances. This substrate is one of the many compounds commonly found on hair (see U. R. Bernier et. al., Anal. Chem. 2000, 72, 747.)

At a skin-relevant pH of 5.5, a significant fluorescence signal is observed due to the action of *Malassezia* on substrate. The oxidative species produced by the cells cause significant fluorescence. This fluorescence is quenched when a reducing species, like ethoxyquin (EQ), is added to the mixture. No fluorescence is observed from the *Malassezia* cells when no substrate is present or from the substrate alone, without the *Malassezia* cells.

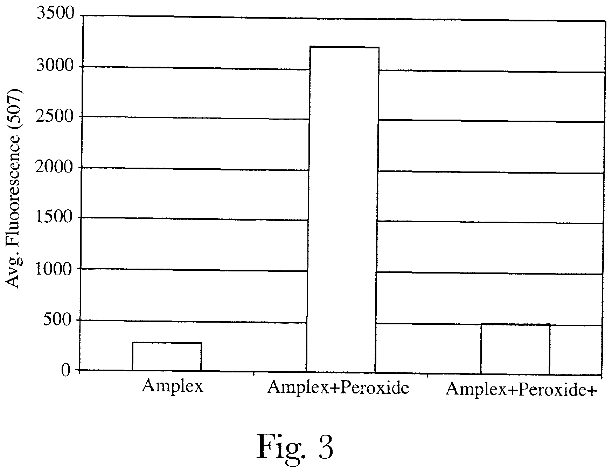

Figure 3 shows that a similar fluorescence signal is produced when 10µM H₂O₂ is manually added to the AMP dye. This signal is also quenched by ethoxyquin.

Other possible mechanisms may cause oxidative damage to the hair, as Malassezia are known to secrete approximately 500 different proteins. (See Xu, J., et al; Proceedings of the National Academy of Science, USA, (2007), 104(47), 18731). Many have unknown function, but some are certainly lipase-like in nature and can damage hair cuticle structure. (See DeAngelis et al; J. Invest. Dermatol. 127, 2138.)

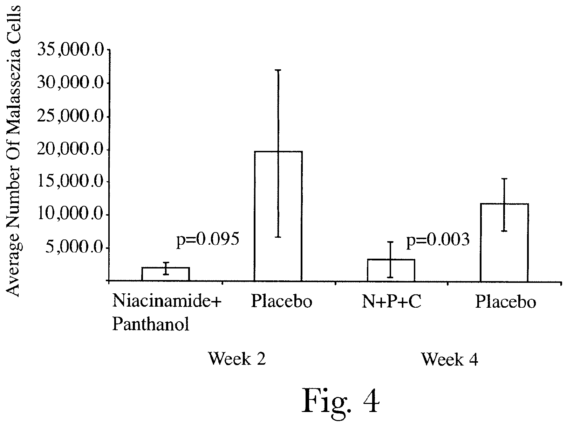

Figure 4 demonstrates the removal of *Malassezia* using FFRAs. The FFRAs demonstrated below include: (1) a mixture of niacinamide, caffeine, and panthenol; and (2) a mixture of niacinamide and panthenol. Figure 4 shows that Malassezia are removed from the scalp by applying these FFRAs to the scalp. In this particular experiment, the products were delivered in a leave-on context.

Figure 4 shows that the average number of *Malassezia* cells on the scalp was reduced significantly (p≤0.095) after 2 to 4 weeks of daily treatment with either of the two formulations, in a 25% alcohol aqueous vehicle. (As used herein, N = niacinamide; P = panthenol; C = caffeine.)

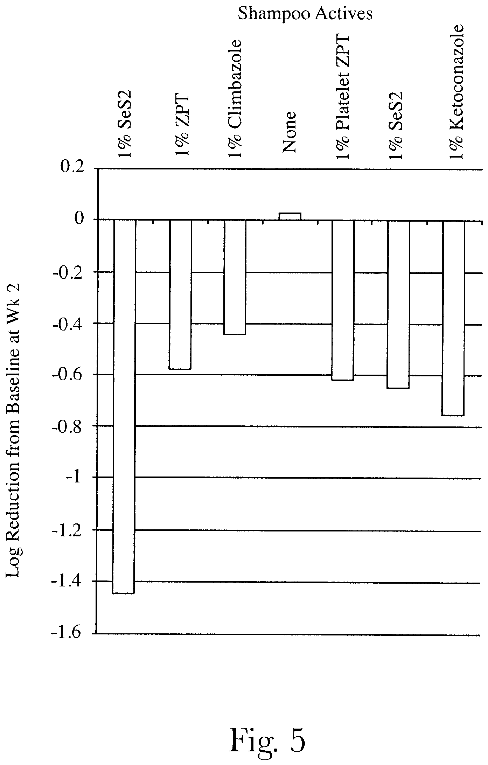

Figure 5 shows that a variety of anti-dandruff actives can also reduce the level of *Malassezia* when delivered from a shampoo formulation context after two weeks of usage.

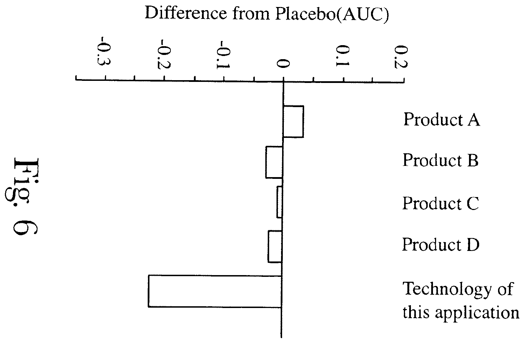

To demonstrate this hypothesis, the present investigators conducted a split-head clinical study, comparing the caffeine/niacinamide/panthenol FFRA formulation (delivered from a tonic) to a variety of in-market products which claim to improve hair quality but do not containing FFRAs. As shown by Figure 6, the FFRA formulation reduced the amount of oxidative damage to the hair caused by *Malassezia.*

Oxidative damage to hair can be measured by examining the ratio of diamond ATR-IR-spectra bands for SO₃= and amide present in the hair. Specifically, the presence of SO₃= is an indicator of oxidative damage in hair. Therefore, a low SO₃=: amide ratio indicates lower oxidative damage. Variables in hair sample size are controlled by normalizing results to the amide content (a relative constant) in the hair sample. Figure 6 shows the SO₃=: amide ratios obtained for hair treated with the claimed technology or commercially available product or placebo. The graph shows the difference between area under the spectra band curves (AUC) for five different treatments used in a split-head study. Treatments A through D are commercially available products that have been reported to improve hair quality. This figure shows that the FFRA combination provides the greatest relative prevention in SO₃= band area when compared to placebo in the split head study. This prevention of oxidative damage is statistically significant at p = 0.0037.

Based upon these results, it is reasonable to conclude that FFRAs can prevent oxidative damage by removing *Malassezia.* Since a wide range of other materials have also been shown to remove *Malassezia* in both a leave-on and shampoo rinse-off context, these actives should also produce a similar prevention of oxidative damage.

### II. FFRA Compositions

Compositions comprising FFRAs can be in any suitable form, such as a liquid, cream, shampoo, conditioner, mousse, or tonic. Although one skilled in the art will be able to determine the appropriate amount of a particular FFRA to include in a particular composition, typical concentrations of FFRAs included in compositions can be 0.1-10%, and in other embodiments from 0.5-5% for rinse off products such as shampoos and conditioners; furthermore, they can be 0.001-0.5%, and in other embodiment from 0.005-0.5%, and in still other embodiments from 0.01-0.1 %, for leave-in treatments such as tonics or mousses.

The FFRA can also be combined with other suitable materials as desired. In a particular embodiment, the FFRA is combined with a material selected from the group consisting of butylated hydroxytoluene (BHT), butylated hydroxyanisole (BHA), xanthines (e.g. caffeine), agmatine, aminoguanidine, ethoxyquin, cetyl pyridinium chloride, green tea extract, catechins, phytosterols, ursolic acid, plant extracts, plant extract compounds, 3-butylidenepthalide, its salts, its derivatives, and mixtures thereof, keratolytic agents (e.g., salicylic acid), and combinations thereof.

### A. Vitamin B₃ Compounds

The compositions of the present invention include an effective amount of a vitamin B₃ compound. Vitamin B₃ compounds include those described in U.S. Patent No. 5,939,082. In particular embodiments, the composition can alternatively comprise from 0.001% to 50%, from 0.01% to 20%, from 0.05% to 10%, from 0.1% to 7%, or from 0.5% to 5%, by weight of the composition, of the vitamin B₃ compound.

As used herein, "vitamin B₃ compound" means a compound having the formula: 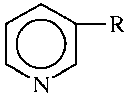 wherein R is - CONH₂ (i.e., niacinamide), - COOH (i.e., nicotinic acid) or - CH2OH (i.e., nicotinyl alcohol); derivatives thereof; and salts of any of the foregoing.

Exemplary derivatives of the foregoing vitamin B3 compounds include nicotinic acid esters, including non-vasodilating esters of nicotinic acid (e.g, tocopherol nicotinate, myristyl nicotinate), nicotinyl amino acids, nicotinyl alcohol esters of carboxylic acids, nicotinic acid N-oxide and niacinamide N-oxide.

Suitable esters of nicotinic acid include nicotinic acid esters of C₁-C₂₂, preferably C₁-C₁₆, more preferably C₁-C₆ alcohols. The alcohols are suitably straight-chain or branched chain, cyclic or acyclic, saturated or unsaturated (including aromatic), and substituted or unsubstituted. The esters are preferably non-vasodilating. As used herein, "non-vasodilating" means that the ester does not commonly yield a visible flushing response after application to the skin in the subject compositions (the majority of the general population would not experience a visible flushing response, although such compounds may cause vasodilation not visible to the naked eye, i.e., the ester is non-rubifacient). Non-vasodilating esters of nicotinic acid include tocopherol nicotinate and inositol hexanicotinate; tocopherol nicotinate is preferred.

Other derivatives of the vitamin B₃ compound are derivatives of niacinamide resulting from substitution of one or more of the amide group hydrogens. Nonlimiting examples of derivatives of niacinamide useful herein include nicotinyl amino acids, derived, for example, from the reaction of an activated nicotinic acid compound (e.g., nicotinic acid azide or nicotinyl chloride) with an amino acid, and nicotinyl alcohol esters of organic carboxylic acids (e.g., C1 - C18). Specific examples of such derivatives include nicotinuric acid (C8H8N2O3) and nicotinyl hydroxamic acid (C6H6N2O2), which have the following chemical structures:
nicotinuric acid: 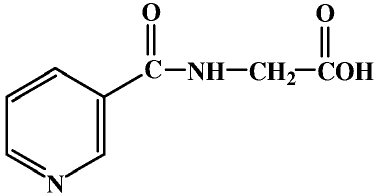
nicotinyl hydroxamic acid:

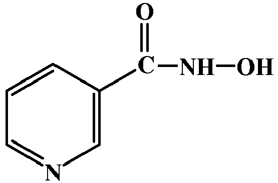

Exemplary nicotinyl alcohol esters include nicotinyl alcohol esters of the carboxylic acids salicylic acid, acetic acid, glycolic acid, palmitic acid and the like. Other non-limiting examples of vitamin B3 compounds useful herein are 2-chloronicotinamide, 6-aminonicotinamide, 6-methylnicotinamide, n-methyl-nicotinamide, n,n-diethylnicotinamide, n-(hydroxymethyl)-nicotinamide, quinolinic acid imide, nicotinanilide, n-benzylnicotinamide, n-ethylnicotinamide, nifenazone, nicotinaldehyde, isonicotinic acid, methyl isonicotinic acid, thionicotinamide, nialamide, 1-(3-pyridylmethyl) urea, 2-mercaptonicotinic acid, nicomol, and niaprazine.

Examples of the above vitamin B₃ compounds are well known in the art and are commercially available from a number of sources, e.g., the Sigma Chemical Company (St. Louis, MO); ICN Biomedicals, Inc. (Irvin, CA) and Aldrich Chemical Company (Milwaukee, WI).

One or more vitamin B₃ compounds may be used herein. Preferred vitamin B3 compounds are niacinamide and tocopherol nicotinate. Niacinamide is more preferred.

When used, salts, derivatives, and salt derivatives of niacinamide are preferably those having substantially the same efficacy as niacinamide.

Salts of the vitamin B3 compound are also useful herein. Nonlimiting examples of salts of the vitamin B3 compound useful herein include organic or inorganic salts, such as inorganic salts with anionic inorganic species (e.g., chloride, bromide, iodide, carbonate, preferably chloride), and organic carboxylic acid salts (including mono-, di- and tri- C1 - C18 carboxylic acid salts, e.g., acetate, salicylate, glycolate, lactate, malate, citrate, preferably monocarboxylic acid salts such as acetate). These and other salts of the vitamin B3 compound can be readily prepared by the skilled artisan, for example, as described by W. Wenner, "The Reaction of L-Ascorbic and D-Iosascorbic Acid with Nicotinic Acid and Its Amide", J. Organic Chemistry, Vol. 14, 22-26 (1949). Wenner describes the synthesis of the ascorbic acid salt of niacinamide.

In a preferred embodiment, the ring nitrogen of the vitamin B₃ compound is substantially chemically free (e.g., unbound and/or unhindered), or after delivery to the skin becomes substantially chemically free ("chemically free" is hereinafter alternatively referred to as "uncomplexed"). More preferably, the vitamin B3 compound is essentially uncomplexed. Therefore, if the composition contains the vitamin B3 compound in a salt or otherwise complexed form, such complex is preferably substantially reversible, more preferably essentially reversible, upon delivery of the composition to the skin. For example, such complex should be substantially reversible at a pH of from about 5.0 to about 6.0. Such reversibility can be readily determined by one having ordinary skill in the art.

More preferably the vitamin B₃ compound is substantially uncomplexed in the composition prior to delivery to the keratinous tissue. Exemplary approaches to minimizing or preventing the formation of undesirable complexes include omission of materials which form substantially irreversible or other complexes with the vitamin B₃ compound, pH adjustment, ionic strength adjustment, the use of surfactants, and formulating wherein the vitamin B₃ compound and materials which complex therewith are in different phases. Such approaches are well within the level of ordinary skill in the art.

Thus, in a preferred embodiment, the vitamin B3 compound contains a limited amount of the salt form and is more preferably substantially free of salts of a vitamin B₃ compound. Preferably the vitamin B₃ compound contains less than about 50% of such salt, and is more preferably essentially free of the salt form. The vitamin B3 compound in the compositions hereof having a pH of from about 4 to about 7 typically contain less than about 50% of the salt form.

The vitamin B₃ compound may be included as the substantially pure material, or as an extract obtained by suitable physical and/or chemical isolation from natural (e.g., plant) sources. The vitamin B₃ compound is preferably substantially pure, more preferably essentially pure.

### B. Panthenol and Pantothenic Acid Derivatives

The compositions of the present invention comprise an effective amount of panthenol and/or pantothenic acid derivatives. Panthenol and its derivatives can include D-panthenol ([R]-2,4-dihydroxy-N-[3-hydroxypropyl)]-3,3-dimethylbutamide), DL-panthenol, pantothenic acids and their salts, preferably the calcium salt, panthenyl triacetate, royal jelly, panthetine, pantotheine, panthenyl ethyl ether, pangamic acid, pantoyl lactose, Vitamin B complex, or mixtures thereof.

Compositions comprising pantothenic acid derivatives that remain more stable than panthenol and other similar materials in acidic compositions or in compositions containing acid-producing materials such as aluminum-containing actives, can also be suitable for use herein. The selected pantothenic acid derivatives are most typically in liquid form and dispersed throughout or otherwise solubilized within the liquid carrier component of the composition.

The term "pantothenic acid derivative" as used herein refers to those materials that conform to the formula: 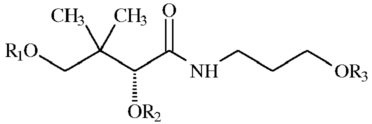 wherein R₁, R₂ and R₃ are hydrogen, C2-C20 hydrocarbons, C2-C20 carboxylic acid esters, or combinations thereof, provided that not more than two of R1, R2 and R3 are hydrogen. In one embodiment, R₁, R₂ and R₃ are independently selected from hydrogen, C2-C8 hydrocarbons, C2-C8 carboxylic acid esters, or combinations thereof; in another embodiment, R₁ and R₂ are hydrogen, and R₃ is a C2-C8 hydrocarbon, C2-C8 carboxylic acid ester, or combinations thereof; in yet another embodiment, R₁ and R₂ are hydrogen and R₃ is ethyl. The selected pantothenic acid derivatives may be derived or otherwise obtained from any known source, which may include pantothenic acid or materials other than pantothenic acid, so long as the resulting material has the above defined chemical formula.

Specific non-limiting examples of selected pantothenic acid derivatives for use herein include ethyl panthenol, panthenyl triacetate, and combinations thereof. In a particular embodiment, a pantothenic acid derivative comprises the d-isomeric form(s) of such derivative form(s), such as d-ethyl panthenol.

In one embodiment, the panthenol and/or pantothenic acid derivative is used, alternatively, in an amount of from 0.01% to 10%, from 0.1% to 5%, or from 0.2% to 3%, by weight of the composition.

### C. Xanthine Compounds

The compositions of the present invention include a xanthine compound. As used herein, "xanthine compound' means one or more xanthines, derivatives therof, and mixtures thereof. Xanthine Compounds that can be useful herein include, but are not limited to, caffeine, xanthine, 1-methyl xanthine, theophylline, theobromine, derivatives thereof, and mixtures thereof. In one embodiment, the composition comprises from about 0.1% to about 10% of a xanthine compound, in another embodiment from about 0.5% to about 5% of a xanthine compound, and in yet another embodiment from about 1% to about 2% of a xanthine compound. The FFRA composition comprises a mixture of a xanthine compound, a vitamin B3 compound, and a panthenol compound. In a particular embodiment, the synergistic mixture comprises caffeine, niacinamide, and panthenol. In one embodiment, the composition comprises from about 0.1% to about 10% of a xanthine compound (e.g., caffeine), in another embodiment from about 0.5% to about 5% of a xanthine compound, and in yet another embodiment from about 1% to about 2% of a xanthine compound. In some embodiments, the composition comprises from about 0.1% to about 25% of a vitamin B3 compound (e.g., niacinamide), in another embodiment from about 0.5% to about 15% of a vitamin B3 compound, and in yet another embodiment from about 3.5% to about 7.5% of a vitamin B3 compound. In some embodiments, the composition comprises from about 0.01% to about 3% of a panthenol compound (e.g., panthenol), in another embodiment from about 0.02% to about 1% of a panthenol compound, and in yet another embodiment from about 0.2% to about 0.5% of a panthenol compound. The composition can optionally comprise any other suitable ingredients as desired. In one embodiment, the composition also comprises a thickener that helps to hold the active agents on the scalp, providing substantivity to the composition, such that it does not drip undesirably onto unintended areas of the body, clothing, or home furnishings.

### II. Methods for Preventing Oxidative Damage to Hair

The present invention also provides a method for preventing oxidative damage of hair, leading to an appearance of shinier, more manageable hair. In one aspect, the method comprises applying a hair care composition comprising a FFRA to a skin surface from which a region of styled hair grows. For instance, the hair care composition can be applied to the scalp and/or face (e.g., beard or moustache area). In another embodiment, the method comprises topically applying a hair care composition comprising an effective amount of a FFRA to a region of skin of a mammal seeking to prevent oxidative damage in hair.

The region of hair can be located on any part of the body. For instance, it can grow from a skin surface located on at least a portion of the scalp or the face or the neck.

In still another embodiment, the method comprises applying the composition according to a regimen, wherein said regimen comprises:
(a) cleansing the scalp and/or face to form a cleansed scalp and/or face;
(b) topically applying the composition to said cleansed scalp and/or cleansed face.

In yet another embodiment, the method comprises:
(a) identifying a region of mammalian hair where prevention of oxidative damage is desired;
(b) topically applying a hair care composition to the region of skin from which said hair grows, wherein said composition comprises an effective amount of an FFRA.

In another aspect, the present invention provides a method for promoting hair shine, comprising:
(a) identifying a region of mammalian hair where hair shine is desired;
(b) topically applying a hair care composition to the region of skin from which said hair grows, wherein said composition comprises an effective amount of an FFRA.

### IV. Method of Marketing

In another aspect, the present invention provides methods of marketing and methods of marketing hair care compositions that can be used to prevent oxidative damage in hair. In one embodiment, the method of marketing comprises:
(1) a container;
(2) a hair care composition contained within said container, wherein said hair care composition comprises a FFRA; and
(3) a communication, wherein said communication communicates that use of said hair care composition can prevent oxidative damage in hair.

In another aspect, the present invention provides methods of marketing hair care compositions that can be used to prevent oxidative damage in hair. In one embodiment, the method comprises:
(a) offering for sale a hair care composition comprising a FFRA;
(b) communicating that said composition can be used to prevent oxidative damage in hair.

In another aspect, the invention provides a marketing method that utilizes a comparison of a first hair care composition to a second hair care composition, in order to market the first hair care composition. In one embodiment, the method comprises offering for sale a first method of marketing, wherein said first method of marketing comprises:
(a) a first hair care composition; and
(b) a communication, wherein said communication compares said first hair care composition to a second hair care composition, wherein said second hair care composition is comprised in a second method of marketing, wherein said second method of marketing comprises:
   (1) said second hair care composition comprising a FFRA; and
   (2) a second communication to a potential consumer, wherein said second communication communicates that said second hair care composition can be used to prevent oxidative damage in hair.

In another aspect, the invention provides a marketing method that utilizes at least one visual cue to communicate that a first hair care composition is similar to or the same as a second hair care composition, in order to market the first hair care composition. In one embodiment, the visual cue comprises a message. In particular embodiments, the message can comprise words such as "compare," "compare to", "like", "similar", "try instead of," or the like. In another embodiment, the visual cue can comprise the same or similar graphics as those included on or near the packaging of the second hair care composition. A visual cue can be located at or on any suitable location. For instance, a visual cue can be located on or near product packaging, or on or near a store shelf.

In a particular embodiment, the first hair care composition is marketed in a container having at least two of the same colors as the container in which the second hair care composition is marketed. In one embodiment, the method comprises a method of marketing a first hair care composition, wherein said method comprises:
(a) offering for sale a first method of marketing, wherein said first method of marketing comprises:
   (1) a first container;
   (2) a first hair care composition contained within said container;
   (3) a first set of graphics disposed upon said first container, wherein said first set of graphics comprises at least two colors;
(b) locating said first method of marketing within visual sight of a second method of marketing, wherein said second method of marketing comprises:
   (1) a second container;
   (2) a second hair care composition contained within said second container,
   (3) a second set of graphics disposed upon said second container, wherein said second set of graphics comprises:
      (i) at least two of the same colors as those colors included in said first set of graphics; and
      (ii) a communication to a potential consumer, wherein said communication informs said potential consumer that said second hair care composition can be used to prevent oxidative damage in hair.

As used herein, the term "potential consumer" means an actual or potential purchaser and/or an actual or potential user of the method of marketing and/or hair care composition.

Any container from which the hair care composition can be stored and/or contained can be used herein. Suitable containers can include, but are not limited to, bottles, tottles, tubes, pouches, boxes, tubs, and cans. Furthermore, containers can include primary containers, which contain the hair care composition itself, or secondary containers, which contain at least one primary container that contains the composition.

As used herein, "set of graphics" or "graphics" refers to the text and/or pictorial images that are disposed on a container. As used herein, "disposed on" means integral with and/or located on the container and can include, but is not limited to, disposed directly thereon (e.g., printed directly on the container), disposed indirectly thereon (e.g., printed on a sticker that is affixed to the outer portion of the container), and/or applied to the container by any other suitable means (e.g., sprayed, bonded, drawn, painted, printed, or molded).

As used herein, "communication" means a message, and can include but is not limited to a printed (e.g., printed material attached directly or indirectly to the container), electronic, or broadcast message.

Optionally, said first method of marketing and said second method of marketing can be located within visual sight of one another. In a particular embodiment, said first method of marketing and said second method of marketing can be located adjacent to one another on a retail shelf or other retail display.

As used herein, "located within visual sight" of one another" means that the first method of marketing and the second method of marketing are located in proximity to one another such that a human with unassisted 20/20 vision can see both the first method of marketing and the second method of marketing at the same time. In a particular embodiment, said first method of marketing and said second method of marketing are located within 2 meters of each other. In another embodiment, said first method of marketing and said second method of marketing are located within 1 meter of each other. In a specific embodiment, said first method of marketing and said second method of marketing are located within 0.5 meter of each other.

As used herein, "similar" means alike in someway. For instance, alike in composition, composition of active ingredients, and/or benefits that can be provided from use of the composition.

### EXAMPLES

The following are non-limiting examples of the present invention. The examples are given solely for the purpose of illustration and are not to be construed as limitations of the present invention, as many variations thereof are possible without departing from the spirit and scope of the invention, which would be recognized by one of ordinary skill in the art.

In the examples, all concentrations are listed as weight percent, unless otherwise specified and may exclude minor materials such as diluents, filler, and so forth. The listed formulations, therefore, comprise the listed components and any minor materials associated with such components. As is apparent to one of ordinary skill in the art, the selection of these minors will vary depending on the physical and chemical characteristics of the particular ingredients selected to make the present invention as described herein.

### Examples 1-4: Shampoo examples

**Table 2**

| **Component** | **1 (wt%)** | **2 (wt%)** | **3 (wt%)** | **4 (wt%)** |
|---|---|---|---|---|
| Water | Q.S. | Q.S. | Q.S. | Q.S. |
| FFRA component | 5% | 1% | 0.1% | 0.01% |
| Rheology modifying system, anionic polymer MVE/MA crosslinked copolymer (Stabileze 06) | 0.0500 | 0.0500 | - | - |
| Rheology modifying system, clay Hydrous Na, Mg silicate (Laponite XLS) | 0.0500 | 0.0500 | 0.05 | - |
| Hydroxypropyl methylcellulose (PrimaFlo) | - | - | 0.1 | - |
| Polyquaternium 10 (Ucare Polymer LR-400) | 0.5000 | 0.5000 | 0.5 | 0.5000 |
| Coconut monoethanolamide (Monamid CMA) | 1.0909 | 1.0286 | 1.0286 | 1.5000 |
| Disodium EDTA (Disslovine Na₂S) | 0.1400 | 0.1400 | 0.14 | 0.0991 |
| Sodium Benzoate (Purox S Grains) | 0.2500 | 0.2500 | 0.25 | 0.2500 |
| Sodium Citrate Dihydrate | 0.4520 | 0.4520 | 0.452 | 0.4520 |
| Sodium Laureth-3- Sulfate (SLE3S) | 2.1818 | - | - | - |
| Cocamidopropyl Betaine (Tegobetaine F-B) | 2.1818 | - | - | - |
| Sodium lauryl sulfate (SLS) | 6.5455 | - | - | - |
| Citric Acid | 0.0781 | - | - | 0.0400 |
| BHT | 0.5000 | 0.5000 | 0.5000 | 0.5000 |
| Sodium Chloride | 0.2500 | 0.7500 | 0.50 | 0.0145 |
| Sodium Hydroxide | 0.0126 | - | - | - |
| Dimethicone (Viscasil 3000,000) | 1.3510 | 1.3510 | 1.3510 | 1.3510 |
| Ammonium Laureth-3-Sulfate (AE3S) | 0.0676 | 4.1143 | 6.00 | 6.0000 |
| Ethylene glycol distearate (EGDS) | 1.5000 | 1.5000 | 1.5 | 1.5000 |
| Ammonium Lauryl Sulfate (ALS) | 1.5000 | 6.8751 | 6.8751 | 10.0000 |
| Hexamidine diisethionate | 0.1000 | 0.1000 | 0.1000 | 0.1000 |
| Glyceryl dilaurate | 2.0000 | 2.0000 | 2.0000 | 2.0000 |
| Methylchloroisothiazolinone & Methylisothiazolinone (Kathon CG) | 0.0005 | 0.0005 | 0.0005 | 0.0005 |
| Fragrance | 0.7000 | 0.7000 | 0.7 | 0.7000 |
| PEG 7M (Polyox WSR-N-750) | 0.1000 | - | - | 0.1000 |
| DL Panthenol 50% soln. (DL-Panthenol 50L) | 0.0300 | 0.0300 | 0.03 | 0.0300 |
| DL Panthenyl Ethyl Ether (Pantyl Ethyl Ether) | 0.0300 | 0.0300 | 0.03 | 0.0300 |
| Lysine Monochloride | 0.0280- | 0.0280 | 0.0280 | 0.0280 |
| L-Tyrosine Methylester Hydrochloride (Methyl Tyrosine) | 0.0138 | 0.0138 | 0.0138 | 0.0138 |
| Histidine | 0.0080 | 0.0080 | 0.0080 | 0.0080 |
| Cetyl Alcohol | ----- | ----- | | 0.9000 |

### Examples 5-7: Conditioner examples

**Table 3**

| **Component** | **5 (wt%)** | **6 (wt%)** | **7 (wt%)** |
|---|---|---|---|
| Dimethicone compound-1 *1 | - | 4.2 | |
| Dimethicone compound-2 *2 | - | - | 2.000 |
| Silicone compound-2 *3 | 3.500 | - | |
| ZPT (FFRA component) *20 | 5% | 1% | 0.1% |
| Behenyl trimethyl ammonium chloride *6 | 2.250 | - | 3.380 |
| Isopropyl alcohol | 0.598 | - | 0.900 |
| Stearamidopropyl dimethylamine *7 | - | 2.000 | - |
| Glutamic acid *8 | - | 0.640 | - |
| Cetyl alcohol *9 | 1.900 | 2.500 | 2.300 |
| Stearyl alcohol *10 | 4.600 | 4.500 | 4.200 |
| BHT | 0.500 | 0.500 | 0.500 |
| Benzyl alcohol | 0.400 | 0.400 | 0.400 |
| Glyceryl dilaurate | 2.000 | 2.000 | 2.000 |
| Methylchloroisothiazolinone/ Methylisothiazolinone *14 | 0.0005 | 0.0005 | 0.0005 |
| Perfume | 0.350 | 0.500 | 0.350 |
| NaOH | 0.014 | - | 0.014 |
| Panthenol *15 | 0.050 | - | 0.05 |
| Panthenyl ethyl ether *16 | 0.050 | - | 0.05 |
| Hexamidine diisethionate | 0.100 | 0.100 | 0.100 |
| Hydrolyzed collagen *17 | - | 0.010 | - |
| Vitamin E *18 | - | 0.010 | - |
| Octyl methoxycinnamate | - | 0.090 | - |
| Benzophenone-3 | - | 0.090 | - |
| Disodium EDTA | 0.127 | 0.127 | 0.127 |
| Deionized water | Qs | Qs | Qs |

| | | | |
|---|---|---|---|
| Definitions of Components: *1 Dimethicone/Cyclomethicone: a blend dimethicone having a viscosity of 18,000,000mPas and cyclopentasiloxane available from GE Toshiba *2 Dimethicone blend: a blend of dimethicone having a viscosity of 18,000,000mPas and dimethicone having a viscosity of 200mPas available from GE Toshiba *3 Available from GE having a viscosity 10,000mPas, and having following formula (I): (R₁)ₐG₃₋ₐ-Si-(-OSiG₂)ₙ-(-OSiG_{b}(R₁)_{2-b})ₘ-O-SiG₃₋ₐ(R₁)ₐ (I) wherein G is methyl; a is an integer of 1; b is 0, 1 or 2, preferably 1; n is a number from 400 to about 600; m is an integer of 0; R₁ is a monovalent radical conforming to the general formula CqH_{2q}L, wherein q is an integer of 3 and L is -N(CH₃)₂ *6 Behenyl trimethyl ammonium chloride/Isopropyl alcohol: Genamin KDMP available from Clariant *7 Stearamidopropyl dimethylamine: Lexamine S-13 available from Inolex *8 Glutamic acid: available from Ajinomoto *9 Cetyl alcohol: Konol series available from Shin Nihon Rika. *10 Stearyl alcohol: Konol series available from Shin Nihon Rika. *11 Polysorbate-20: Glycosperse L-20K available from Lonza Inc. *12 PPG-34: New Pol PP-2000 available from Sanyo Kasei. *13 Polyalphaolefin: PureSyn 100 available from ExxonMobil Chemical Company *14 Methylchloroisothiazolinone/Methylisothiazolinone: Kathon CG available from Rohm & Haas *15 Panthenol: Available from Roche. *16 Panthenyl ethyl ether: Available from Roche. *17 Hydrolyzed collagen: Peptein 2000 available from Hormel. *18 Vitamin E: Emix-d available from Eisai. *19 Decyl glucoside: Plantacare 2000UP available from Cognis Japan Ltd. *20 FFRA component | | | |

### Example 8: Tonic example

**Table 4**

| **Component** | **8 (wt%)** |
|---|---|
| Alcohol 100% DEB 100 (Ethanol) | 25.000 |
| Carbomer (Carbopol Ultrez 10) | 0.100 |
| Hexamidine diisethionate | 0.100 |
| Glyceryl dilaurate | 2.000 |
| BHT | 0.500 |
| Triethanolamine | 0.200 |
| Caffeine | 1.500 |
| Niacinamide | 5.000 |
| Panthenol | 0.300 |
| Deionized water | Qs |

| | |
|---|---|
| *20-FFRA in this example is the combination of niacinamide, panthenol, and caffeine | |

### Example 9: Dye Example

**Table 5**

| **Component** | **9 (wt%)** |
|---|---|
| Propylene glycol | 9.500 |
| Ammonium hydroxide | 5.000 |
| Ethoxydiglycol | 4.000 |
| Ethanolamine | 4.500 |
| Oleic acid | 1.000 |
| Hexylene glycol | 6.000 |
| Hexamidine diisethionate | 0.100 |
| Glyceryl dilaurate | 2.000 |
| BHT | 0.500 |
| Cocamidopropyl betaine | 3.500 |
| Oleth-10 | 0.300 |
| Oleth-2 | 0.300 |
| Dilinoleic acid | 1.500 |
| C12-C15 Pareth-3 | 0.500 |
| Soytrimonium chloride | 7.000 |
| Sodium metasilicate | 0.050 |
| Erythorbic acid | 0.500 |
| EDTA | 0.030 |
| Sodium sulfite | 0.300 |
| 1-Phenyl-3-methyl-5-pyrazolone | 0.200 |
| Deionized water | Qs |
| 1% Ketokonazole - *20 (FFRA) | 1% |

### Example 10: Mousse

**Table 6**

| **Component** | **10 (wt%)** |
|---|---|
| Ethanol | 51.800 |
| Propylene glycol | 5.000 |
| Propellant P75 | 4.300 |
| Cetyl alcohol | 2.200 |
| Glyceryl dilaurate | 2.000 |
| Stearyl alcohol | 1.000 |
| Polyoxyethylene lauryl alcohol | 1.000 |
| BHT | 0.500 |
| Polysorbate 60 | 0.400 |
| Hexamidine diisethionate | 0.100 |
| Caffeine | 1.500 |
| Niacinamide | 5.000 |
| Panthenol | 0.300 |
| Acetic acid | Qs pH 6.0 |
| Deionized water | Qs |

| | |
|---|---|
| *20-FFRA in this example is the combination of niacinamide, panthenol, and caffeine | |

### Example 11: Method of Marketing

The shampoo of Example 1 is packaged into a blue and white container and offered for sale to consumers at a retail store. A label on the container communicates that when this shampoo is used to wash hair, it will help to prevent oxidative damage in hair.

### Example 12: Method of Marketing

A shampoo contained in a blue and white bottle (herein "Subject Shampoo") is located on a shelf next to the shampoo of Example 11. A label is attached to the Subject Shampoo's bottle which directs the consumer to compare the Subject Shampoo to the shampoo of Example 11.

### Example 13: Clinical Study

In a clinical study, the researchers collected hairs at Week 12 and assessed oxidative damage by measuring the ratio of cysteic acid (SO3=)/Amide using ATR-IR. The passages below explain the clinical and the data. The next section describes ATR-IR as a valid method to assess oxidative damage.

This was a fourteen week, double blinded, randomized, split-head and controlled clinical study in women, ages 18-65 (inclusive) who perceived themselves as having thinning hair. This study consisted of a 2 week pre-treatment phase followed by a 12 week treatment phase.

Hair samples were collected by plucking hairs proximal to the imaging site at Baseline and Week 12 from each side of the head and sent to the researchers. Upon arrival, samples were processed using ATR-IR to determine the level of oxidative damage (as measured by level of cysteic acid) at the beginning and end of treatment. Treatment product was compared to placebo to determine any beneficial effects from the treatment leg.

Levels of cysteic acid are measured by ATR-IR (Attenuated Total Reflectance-Fourier Transform Infra-Red). For each hair fiber, the level of cysteic acid (oxidation product) was determined from 1, 3, 5, 7, 10, and 50mm distal from the inner root sheath at Week 12. Measurements taken from 1-10mm distal from the inner root sheath represent new growth of hair during the clinical trial. All cysteic acid levels/hair were normalized to protein concentration (Amide). Simplistically, ATR-IR is a microscope that emits infra-red and analyzes the reflectance from the hair. The operator is able to focus on the specific region of interest on the surface of the hair, to measure the amount of cysteic acid (SO3= and Amide) from precise areas along the hair fiber. The resultant data is expressed and SO3=/Amide; the higher the number, the higher the ratio of SO3=/Amide (less SO3=/more Amide) and thus, the greater the oxidative damage.

As demonstrated by Figure 7, statistically significantly less oxidative damage was observed with the Experimental (Test) Product (which is a combination of caffeine, niacinamide and panthenol) versus the Placebo Product. Test Product is able to retard the level of oxidative damage when moving from the root to the tip of the hair fiber. Additionally, there is a positive correlation between the level of oxidation and the distance from the root in the Test Product Placebo leg.

Oxidation results between Test Product and Placebo at Week 12 is shown in Table 7. A higher ratio (larger number) indicates a greater level of oxidative damage.

**Table 7**

| N | Treatment | Adjusted Mean SO3=/Amide Ratio |
|---|---|---|
| 70 | Test Product Placebo | 0.401 A |
| 70 | Test Product | 0.228 B |

Alpha characters denote significance of p<0.01

The measurement of disulfide oxidation was measured using Attenuated Total Reflectance-Fourier Transform Infra-Red (ATR-IR) fitted with a diamond crystal. This employs the use of infra-red, at specific infra-red absorptions to target cysteic acid using a microscope setup. This system allows the assessment of oxidative damage on the surface of the hair, at specific points along the hair shaft.

FT-IR spectroscopy in attenuated total reflectance (ATR) mode has been mainly used to study the oxidation of hair and, more precisely, its photodamage and the photoprotection afforded by different ingredients. Cysteic acid and other cystine oxides give well-defined absorption peaks. Diamond cell ATR, which enables to apply higher pressure on the sample and thus contributes to a better contact, provided more reproducible results than conventional ATR crystal. (The Science of Hair Care, 2nd edition. Edited by Claude Bouillon and John Wilkinson. Pg 421) The microscope technique is the most useful method as reproducible spectra with good transmission ranges and reduced noise levels are obtained. This technique is particularly suitable when small areas of a fiber are to be examined. The use of second order derivative spectroscopy allows small changes in oxidative damage to be assessed and gives an opportunity for the quantitative analyses of sulfur oxidation products of cystine. (Joy-M and Lewis-DM. The use of Fourier transform infra-red spectroscopy I the study of the surface chemistry of hair fibers.
International Journal of Cosmetic Science 13, 249-261 (1991))

These results demonstrate an increase in oxidation of cystine to cysteic acid as measurements are taken progressively distal from the scalp along the hair fiber in the Test Product Placebo leg. These results mimic those found in externally published results on the effects of natural weathering. (Comparison of root end and tip end of hair allows one to examine the effects of natural weathering. Cysteic acid was found to increase going from root to tip, although the degree of increase was found to be quite variable. As weathering (exposure to sunlight, wind and grooming, etc.) increases, the intensity of the cysteic acid band at 1040 cm⁻¹ increases. (Martin-K. 4. Infrared and Raman Studies of Skin and Hair: A review of cosmetic spectroscopy. The Internet Journal of Vibrational Spectroscopy. Volume 3, Edition 2 (1999)-Unilever) Results indicate that the concentration of cysteic acid in natural untreated hair increases from root to tip. (Joy-M and Lewis-DM. The use of Fourier transform infra-red spectroscopy I the study of the surface chemistry of hair fibres. International Journal of Cosmetic Science 13, 249-261 (1991))

In summary, this clinical study shows that the test formulation of Caffeine/Niacinamide/Panthenol reduces the oxidative damage to the hair. This is supported by: (1) Test Product significantly decreased the level of oxidation vs. placebo in a human clinical study, (2) The method used to measure the level of oxidation (ATR-IR) is a well recognized method, and (3) the observation of increased oxidation along the hair shaft from root to tip agrees with the published literature.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean"about 40 mm".

## Claims

1. A non-therapeutic method for preventing oxidative damage of hair, comprising:
a. identifying a region of mammalian hair where prevention of oxidative damage from the presence of fungi in the hair follicule is desired
b. topically applying a hair care composition to the region of skin from which said hair grows, wherein said composition comprises an effective amount of a follicular fungi reduction agent (FFRA), wherein the FFRA composition comprises a mixture of a xanthine compound, a vitamin B₃ compound as FFRA and a panthenol compound as FFRA; and
wherein the composition comprises from 0.1-10% of the xanthine compound, from 0.1 to 25% of the vitamin B₃ compound, and 0.01-3% of the panthenol compound; and
wherein the number of fungi in the hair follicule and surface is decreased.

2. A method according to claim 1, wherein the vitamin B3 compound is a compound having the formula 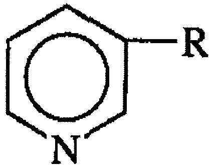 wherein R is - CONH₂, - COOH or - CH₂0H, derivatives thereof, and salts of any of the foregoing, and mixtures thereof; preferably wherein the vitamin B₃ compound includes niacinamide, nicotinic acid, nicotinyl alcohol, nicotinic acid esters, nicotinyl amino acids, nicotinyl alcohol esters of carboxylic acis, nicotinic acid N-oxide, niacinamide N-oxide, derivatives thereof, salts thereof, and mixtures thereof; more preferably wherein the vitamin B₃ compound includes niacinamide, tocopherol nicotinate, or mixture thereof; still more preferably wherein the vitamin B3 compound is niacinamide.

3. A method according to any preceding claim, wherein the composition comprises from 0.1% to 25%, preferably from 0.5% to 15%, more preferably from 3.5% to 7.5%, of the vitamin B₃ compound, by total weight of the composition.

4. A method according to any preceding claim, wherein the panthenol compound comprises panthenol, pantothenic acid, their derivatives, their salts, and mixtures thereof; preferably wherein the panthenol compound includes panthenol, DL-panthenol, panthenyl triacetate, panthetine, pantotheine, panthenyl ethyl ether, pangamic acid, pantoyl lactose, ethyl panthenol, and mixtures thereof; more preferably wherein the panthenol compound is panthenol.

5. A method according to any preceding claim, wherein the composition comprises from 0.01 % to 3%, preferably from 0.02% to 1%, more preferably 0.2% to 0.5%, of the panthenol compound, by total weight of the composition.

6. A method according to any preceding claim, wherein the xanthine compound is one or more xanthines, derivatives thereof, and mixtures thereof; preferably wherein the xanthine compound is caffeine, xanthine, 1-methyl xanthine, theophylline, theobromine, derivatives thereof, and mixtures thereof; more preferably the xanthine compound is caffeine.

7. A method according to any preceding claim, wherein the composition comprises from 0.1% to 10%, preferably from 0.5% to 5%, more preferably from 1% to 2%, of a xanthine compound, by total weight of the composition.

8. A method according to any preceding claim, wherein the vitamin B3 compound is niacinamide, the panthenol compound is panthenol, and the xanthine compound is caffeine.

9. A method according to any preceding claim, wherein the composition comprises a dermatogically-acceptable carrier.

10. A method according to any preceding claim, wherein the composition is in a form of a liquid, cream, shampoo, conditioner, mouse or tonic.

11. Non-therapeutic use of a composition, as defined in any preceding claim, for preventing oxidative damage of hair attributed to the presence of fungi in the hair follicule; more preferably for preventing oxidative damage of hair attributed to the presence of *Malassezia* fungi in the hair follicule; still more preferably for preventing oxidative damage of hair attributed to the presence of *Malassezia* fungi on the hair in the infundibulum region.

12. Non-therapeutic use of an effective amount of a follicular fungi reduction agent (FFRA) composition, said FFRA composition comprising a xanthine compound, a vitamin B₃ compound as FFRA and a panthenol compound as FFRA, for preventing oxidative damage of hair attributed to the presence of fungi in the hair follicule; more preferably for preventing oxidative damage of hair attributed to the presence of *Malassezia* fungi in the hair follicule, still more preferably for preventing oxidative damage of hair attributed to the presence of *Malassezia* fungi on the hair in the infundibulum region.

## Patentansprüche

1. Nichttherapeutisches Verfahren zum Verhindern von oxidativen Schäden am Haar, umfassend:
a. Festlegen eines Bereichs von Säugetierhaar, in dem das Verhindern von oxidativen Schäden durch Vorhandensein von Pilzbefall im Haarfollikel erwünscht ist;
b. utopisches Auftragen einer Haarpflegezusammensetzung auf den Bereich der Haut, auf dem das Haar wächst, wobei die Zusammensetzung eine wirksame Menge Follikelpilzreduktionsmittel (follicular fungi reduction agent, FFRA) umfasst, wobei die FFRA-Zusammensetzung eine Mischung einer Xanthinverbindung, einer Vitamin B₃-Verbindung als FFRA und einer Panthenol-Verbindung als FFRA umfasst; und
wobei die Zusammensetzung von 0,1 bis 10 % der Xanthin-Verbindung, von 0,1 bis 25 % der Vitamin B₃-Verbindung und von 0,01 bis 3 % der Panthenol-Verbindung umfasst; und wobei die Anzahl an Pilz im Haarfollikel und der Haaroberfläche reduziert wird.

2. Verfahren nach Anspruch 1, wobei die Vitamin B₃-Verbindung eine Verbindung folgender Formel ist: 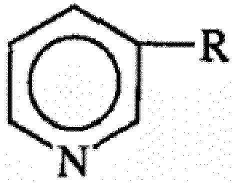 wobei R - CONH₂, -COOH oder CH₂0H ist, Derivate davon und Salze eines der Vorgenannten und Mischungen davon; wobei vorzugsweise die Vitamin B₃-Verbindung Niacinamid, Nikotinsäure, Nikotinylalkohol, Nikotinsäureester, Nikotinylaminosäuren, Nikotinylalkoholester von Carboxylsäuren, Nikotinsäure-N-Oxid, Niacinamid-N-Oxid, Derivate davon, Salze davon und Mischungen davon ist; wobei mehr bevorzugt die Vitamin B₃-Verbindung Niacinamid, Tocopherolnikotinat oder Mischungen davon umfasst; wobei noch mehr bevorzugt die Vitamin B₃-Verbindung Niacinamid ist.

3. Verfahren nach einem der vorstehenden Ansprüche, wobei die Zusammensetzung von 0,1 % bis 25 %, vorzugsweise zu 0,5 % bis 15 %, mehr bevorzugt von 3,5 % bis 7,5 % die Vitamin B₃-Verbindung nach Gesamtgewicht der Zusammensetzung umfasst.

4. Verfahren nach einem der vorstehenden Ansprüche, wobei die Panthenolverbindung Panthenol, Pantothensäure, deren Derivate, deren Salze und Mischungen davon umfasst; wobei vorzugsweise die Panthenolverbindung Panthenol, DL-Panthenol, Panthenyltriacetat, Panthetin, Pantothein, Panthenylethylether, Pangamsäure, Pantoyllactose, Ethylpanthenol und Mischungen davon einschließt; wobei mehr bevorzugt die Panthenolverbindung Panthenol ist.

5. Verfahren nach einem der vorstehenden Ansprüche, wobei die Zusammensetzung von 0,01 % bis 3 %, vorzugsweise von 0,02 % bis 1 %, mehr bevorzugt von 0,2 % bis 0,5 % die Panthenolverbindung nach Gesamtgewicht der Zusammensetzung umfasst.

6. Verfahren nach einem der vorstehenden Ansprüche, wobei die Xanthinverbindung ein oder mehrere Xanthine, Derivate davon und Mischungen davon ist; wobei vorzugsweise die Xanthinverbindung Koffein, Xanthin, 1-Methylxanthin, Theophyllin, Theobromin, Derivate davon und Mischungen davon ist, mehr bevorzugt ist die Xanthinverbindung Koffein.

7. Verfahren nach einem der vorstehenden Ansprüche, wobei die Zusammensetzung von 0,1 % bis 10 %, vorzugsweise von 0,5 % bis 5 %, mehr bevorzugt von 1 % bis 2 % eine Xanthinverbindung nach Gesamtgewicht der Zusammensetzung umfasst.

8. Verfahren nach einem der vorstehenden Ansprüche, wobei die Vitamin B3-Verbindung Niacinamid, die Panthenolverbindung Panthenol und die Xanthinverbindung Koffein ist.

9. Verfahren nach einem der vorstehenden Ansprüche, wobei die Zusammensetzung einen dermatologisch akzeptablen Träger umfasst.

10. Verfahren nach einem der vorstehenden Ansprüche, wobei die Zusammensetzung in Form einer Flüssigkeit, Creme, eines Shampoos, einer Spülung, eines Schaums oder eines Tonics vorliegt.

11. Nichttherapeutische Verwendung einer Zusammensetzung, wie in einem der vorstehenden Ansprüche definiert, zum Verhindern von oxidativen Schäden am Haar, das auf das Vorhandensein von Pilzen im Haarfollikel zurückzuführen ist; mehr bevorzugt zum Verhindern von oxidativen Schäden am Haar aufgrund des Vorhandenseins von Malassezia-Pilzbefall im Haarfollikel; noch mehr bevorzugt zum Verhindern von oxidativen Schäden am Haar, das auf das Vorhandensein von Malassezia-Pilzbefall im Haar im Haarbalgtrichterbereich zurückzuführen ist.

12. Nichttherapeutische Verwendung einer wirksamen Menge Follikelpilzreduktionsmittel (FFRA)-Zusammensetzung, wobei die FFRA-Zusammensetzung eine Xanthinverbindung, eine Vitamin B₃-Verbindung als FFRA und eine Panthenolverbindung als FFRA umfasst, zum Verhindern von oxidativen Schäden am Haar, das auf das Vorhandensein von Pilzbefall im Haarfollikel zurückzuführen ist; mehr bevorzugt zum Verhindern von oxidativen Schäden am Haar aufgrund des Vorhandenseins von Malassezia-Pilzbefall im Haarfollikel, noch mehr bevorzugt zum Verhindern von oxidativen Schäden am Haar, das auf das Vorhandensein von Malassezia-Pilzbefall im Haar im Haarbalgtrichterbereich zurückzuführen ist.

## Revendications

1. Procédé non thérapeutique de prévention d'un dommage oxydatif de cheveux/poils, comprenant :
a. l'identification d'une région de cheveux/poils de mammifères où une prévention de dommage oxydatif par la présence de champignons dans le follicule pileux est souhaitée ;
b. l'application topique d'une composition pour soins des cheveux/poils sur la région de peau à partir de laquelle poussent lesdits poils/cheveux, dans lequel ladite composition comprend une quantité efficace d'un agent de réduction de champignons folliculaires (FFRA), dans lequel la composition de FFRA comprend un mélange d'un composé de xanthine, d'un composé de vitamine B₃ en tant que FFRA et d'un composé de panthénol en tant que FFRA ; et
dans lequel la composition comprend de 0,1 à 10 % du composé de xanthine, de 0,1 à 25 % du composé de vitamine B₃, et de 0,01 à 3 % du composé de panthénol ; et dans lequel le nombre de champignons dans le follicule pileux et la surface pileuse est diminué.

2. Procédé selon la revendication 1, dans lequel le composé de vitamine B3 est un composé de formule 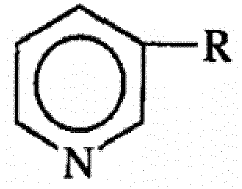 dans lequel R est - CONH₂, -COOH ou CH₂OH, leurs dérivés, et des sels de n'importe quels composés qui précèdent, et leurs mélanges ; de préférence dans lequel le composé de vitamine B₃ inclut du niacinamide, de l'acide nicotinique, de l'alcool nicotinylique, des esters d'acide nicotinique, des acides aminés de nicotinyle, des esters d'alcool nicotinylique d'acides carboxyliques, un N-oxyde d'acide nicotinique, un N-oxyde de niacinamide, leurs dérivés, leurs sels, et leurs mélanges ; plus préférablement dans lequel le composé de vitamine B₃ inclut du niacinamide, du nicotinate de tocophérol, ou un mélange de ceux-ci ; encore plus préférablement dans lequel le composé de vitamine B3 est le niacinamide.

3. Procédé selon l'une quelconque des revendications précédentes, dans lequel la composition comprend de 0,1 % à 25 %, de préférence de 0,5 % à 15 %, plus préférablement de 3,5 % à 7,5 %, du composé de vitamine B₃, en poids total de la composition.

4. Procédé selon l'une quelconque des revendications précédentes, dans lequel le composé de panthénol comprend du panthénol, de l'acide pantothénique, leurs dérivés, leurs sels, et des mélanges de ceux-ci ; de préférence dans lequel le composé de panthénol inclut du panthénol, du DL-panthénol, du triacétate de panthényle, de la panthétine, de la pantothéine, de l'éther éthylique de panthényle, de l'acide pangamique, du pantoyl-lactose, de l'éthyl-panthénol, et leurs mélanges ; plus préférablement dans lequel le composé de panthénol est le panthénol.

5. Procédé selon l'une quelconque des revendications précédentes, dans lequel la composition comprend de 0,01 % à 3 %, de préférence de 0,02 % à 1 %, plus préférablement 0,2 % à 0,5 %, du composé de panthénol, en poids total de la composition.

6. Procédé selon l'une quelconque des revendications précédentes, dans lequel le composé de xanthine est une ou plusieurs xanthines, leurs dérivés, et leurs mélanges ; de préférence dans lequel le composé de xanthine est la caféine, la xanthine, la 1-méthyl-xanthine, la théophylline, la théobromine, leurs dérivés, et leurs mélanges, plus préférablement le composé de xanthine est la caféine.

7. Procédé selon l'une quelconque des revendications précédentes, dans lequel la composition comprend de 0,1 % à 10 %, de préférence de 0,5 % à 5 %, plus préférablement de 1 % à 2 %, d'un composé de xanthine, en poids total de la composition.

8. Procédé selon l'une quelconque des revendications précédentes, dans lequel le composé de vitamine B3 est le niacinamide, le composé de panthénol est le panthénol, et le composé de xanthine est la caféine.

9. Procédé selon l'une quelconque des revendications précédentes, dans lequel la composition comprend un véhicule acceptable sur le plan dermatologique.

10. Procédé selon l'une quelconque des revendications précédentes, dans lequel la composition est sous une forme d'un liquide, d'une crème, d'un shampooing, d'un conditionneur, d'une mousse ou d'un tonic.

11. Utilisation non thérapeutique d'une composition, selon l'une quelconque des revendications précédentes, pour la prévention d'un dommage oxydatif de poils/cheveux attribué à la présence de champignons dans le follicule pileux ; plus préférablement pour la prévention d'un dommage oxydatif de poils/cheveux attribué à la présence de champignons *Malassezia* dans le follicule pileux ; encore plus préférablement pour la prévention d'un dommage oxydatif de poils/cheveux attribué à la présence de champignons *Malassezia* sur les poils/cheveux dans la région de l'infundibulum.

12. Utilisation non thérapeutique d'une quantité efficace d'une composition d'agent de réduction de champignons folliculaires (FFRA), ladite composition de FFRA comprenant un composé de xanthine, un composé de vitamine B₃ en tant que FFRA et un composé de panthénol en tant que FFRA, pour la prévention d'un dommage oxydatif de poils/cheveux attribué à la présence de champignons dans le follicule pileux ; plus préférablement pour la prévention d'un dommage oxydatif de poils/cheveux attribué à la présence de champignons *Malassezia* dans le follicule pileux, encore plus préférablement pour la prévention d'un dommage oxydatif de poils/cheveux attribué à la présence de champignons *Malassezia* sur les poils/cheveux dans la région de l'infundibulum.
